# EUROPEAN PATENT APPLICATION

(11) **EP 2 095 816 A1**
(43) Date of publication of application: **02.09.2009**
(21) Application number: 08003807.8
(22) Date of filing: 29.02.2008
(51) Int. Cl.: A61K 9/72, A61K 31/4196, A61K 31/496, A61K 31/495, A61K 31/415, A61K 31/4174, A61K 9/14

(54) **Nanosuspension with antifungal medication to be administered via inhalation with improved impurity profile and safety**

(71) Applicant: Schlichthaar, Rainer, Dr., 39108 Magdeburg (DE)
(72) Inventor: The designation of the inventor has not yet been filed
(74) Representative: Weiss, Wolfgang

(57) **Abstract**

The present invention is directed to new nanosuspensions of antifungal azole derivatives, particularly itraconazole, with improved impurity profile optimized for inhaled administration for the prevention, reversal and medical treatment of fungal infections of the respiratory tract including adjacent lymph nodes. The new formulation which is devoid of particulate inorganic contamination can be safely administered by inhalation. This administration route results in an improved therapeutic effect and reduced side effect profile as compared to the previously used clinical administration route, i.e. oral or parenteral (intravenous) administration.

## Description

### Field of the Invention

The present invention is directed to the prevention, reversal and medical treatment of fungal infections of the respiratory tract including adjacent lymph nodes using a new nanosuspension formulation to administer antifungal medication via inhalation. The formulation is optimized for the safe inhalation of antifungal azole derivatives, particularly itraconazole. The new highly pure formulation results in an improved therapeutic effect and reduced side effect profile as compared to the previously used clinical administration route, i.e. oral or parenteral (intravenous) administration and especially as compared to the previously described nanosuspensions which are not optimized for inhaled administration. The present invention is directed to a new galenical formulation with improved purity which can be administered via the inhaled route. Due to the selected production process, the compound is not only highly potent but it is especially more safe due to the improved impurity profile. Further more, the manufacturing process is optimized and allows the production of highly concentrated nanosuspensions without anorganic contamination allowing easy high dose inhaled administration even in disabled or unconscious patients or very young patients normally not capable of actively inhaling drugs.

### Background of the invention

Most fungi are opportunistic infectious agents. This does mean that particularly in a situation where other circumstances are supportive to increase the risk for infections, a fungal infection takes place. Thus, for the most part, mycoses are caused by organisms of low pathogenicity emerging as opportunistic organisms thriving in a compromised host. The main factor supporting systemic mycoses is an immune system which cannot successfully fight the pathogenic agent, i.e. a compromised and/or suppressed immune system. While generally a chronic disease may result in a compromised immune response, specific infections are known to reduce the immune response strongly. Especially HIV infections are known to increase the risk for fungal infections. Indeed, fungal infections are the cause of 60% of HIV/AIDS related deaths, according to Dixon et al: Fungal infections: a growing threat (Public Health Rep. 1996 May-Jun;111:226-35.).

Modern medicine has also largely increased the number of immune-compromised or immune-suppressed patients. In general, patients approaching organ transplantation or having received organ transplantation need potent and effective immune suppressive medication. Also, many types of cancer medication interfere with the immune reaction resulting in an increased risk for fungal infections. In case of a fungal pulmonary infection in these patients, the mortality rate approaches 50% (Lin et al., Improving efficacy of antifungal therapy by polymerase chain reaction-based strategy among febrile patients with neutropenia and cancer. Clin Infect Dis. 2001 33:1621-7).

Thus, fungal infections most commonly occur in immune-compromised patients, including those afflicted with AIDS, cancer, leukemia, and other lymphoreticular neoplasms or patients with chronic systemic diseases, including diabetes mellitus and severe pulmonary diseases.

A very delicate patient group are newborn children with a very low birth weight which are also immune-compromised and may experience pulmonary fungal infections.

Again a different population in need of effective anti-fungal medication are patients suffering from cystic fibrosis. Nearly half of all cystic fibrosis patients have a fungus known as Aspergillus fumigatus in their sputum, and about 10% develop a condition known as allergic bronchopulmonary aspergillosis and are in need of anti-fungal treatment.

Another pre-disposing factor supporting fungal infections is the chronic treatment of patients with corticosteroids. Indeed, patients suffering from asthma or chronic obstructive pulmonary disease (COPD) treated with inhaled corticosteroids or high dose oral steroids often suffer from pharyngeal fungus infections and from fungus infections of other organ systems including the lung.

In addition to these factors pre-disposing the occurrence of fungal infections of different organ systems, such infections may also occur due to other (often unknown) causes, such as genetic pre-dispositions. Organ mycoses including lung mycoses can also be found without any obvious pre-disposing factor. Here, we are focussing on fungus infections of the respiratory tract, mainly the nasal and oral cavity, the pharynx, larynx and the lung with adjacent lung lymph nodes.

The respiratory tract including the adjacent cavities, i.e. nasal and oral cavity, pharynx and larynx is a common entrance site for fungal infections since fungal spores are inhaled and the barrier function of the lung epithelium and the mucous membranes of the adjacent surfaces is limited. Furthermore, the high moisture of these surfaces presents an optimal environment for the fungal spores. Typical germs found are Aspergillus species, Histoplasma species, Candida species resulting in aspergillosis, histoplasmosis and candidosis. Other fungi include Pneumocystis pneumonitis which was for example found in pediatric cancer patients, cryptococcal pneumonia infections, Scedosporium prolificans (S. inflatum) infection which was reported after single-lung transplantation, Cunninghamella bertholletiae as an uncommon agent of opportunistic fungal organ infections and blastomycosis which was reported as pulmonary and pleural manifestations as well as onychomycosis. Other fungi may also function as opportunistic infectious agents. Although specific fungi are characteristically found in designated endemic areas, the diseases may surface in remote areas in persons who have recently travelled through the endemic sites. The pathologic picture can be confusing, and pseudoepitheliomatous changes at times resemble malignancy.

The costs of antifungal treatment are huge. In 1998, the costs in the US amounted to 2.6 billion US$ with treatment costs as high 31,200 US$ per patient (Wilson et al., The direct cost and incidence of systemic fungal infections. Value Health. 2002 5:26-34).

According to a recent study published on the web, fungal infections often result in extended hospital stays and are a significant economic burden. Economic analyses show that patients with this type of infection average 17 days in the hospital with mean treatment costs of $62,426. The overall case fatality rate for invasive aspergillosis is estimated to be 58 percent, but approaches 90-100 percent in patients where the infection has spread beyond the initial site (Medical News Today, Public Health News,02 Jun 2005, http://www.medicalnewstoday.com/medicalnews.php?newsid=25481).

### Current treatment and limitations thereof

In many cases, fungal infections are treated with amphotericin B. The compound, being not well orally bioavailable, is administered via different routes including the intravenous and inhaled route. However, amphotericin B is not well tolerated and several germs are or have become resistant to amphothericin B. Currently, amphotericin B still remains the agent of choice for the initial therapy of invasive aspergillosis, although a 1990 review of the literature by Denning and Stevens showed that the overall response rate was only 55% (Denning, D. W., and D. A. Stevens. 1990. Rev. Infect. Dis. 12:1147-1201). Moreover, the therapeutic response to amphotericin B in immunocompromised patients is generally poor (Bennett, J. E. 1995. Aspergillus species, p. 2306-2311. In G. L. Mandell, J. E. Bennett, and R. Dolin (ed.), Principles and practice of infectious diseases, 4th ed. Churchill Livingstone, Inc., New York, N.Y.).

The introduction of the newer orally available azole drugs (ketoconazole, itraconazole and fluconazole, among others) has largely improved the treatment of fungal infections. Ketoconazole has been proven efficacious in certain fungal infections. Itraconazole was found to have a broader spectrum of anti-fungal activity. Finally, fluconazole, taken orally, can also be used but with little advantage and flucytosine has a high rate of secondary tolerance limiting its use.

While systemic administration of the above mentioned drugs, especially the azole derivatives, is effective in the treatment of fungal infections of the respiratory tract, the treatment is afflicted with many problems due to the limited tolerability and due to the high occurrence of side effects. For example, the following direct side effects have been reported for the azole derivatives such as itraconazole and ketoconazole: Nausea, emesis, hypertonia, hypopokaliemia (reduced plasma potassium levels), headache, and increased formation of edema. Cutaneous reactions resembling rush or allergy like reactions, and CNS related symptoms like vertigo and dissorientation were also frequently reported. The most severe side effect which likely is dose dependent is a severe hepatotoxicity which may result in hepatitis, full hepatic failure and liver cirrhosis. The rate of liver toxicity may be as high as 20% of patients. Also toxic effects on other organ systems including the urogenital system have been reported. Other reported severe side effects include rhabdomyolysis and effects on lipid metabolism.

The high metabolic rate which is found to be variable and the non-linear kinetics indicating saturation of metabolic pathways and metabolic enzyme inhibition further complicates the treatment of patients with azole derivatives.

Individual azoles were also found to have specific toxic problems. Absorption of itraconazole is unreliable in seriously ill patients with disturbed gastrointestinal function. Ketoconazole for example was found to reversibly inhibit testosterone production resulting in impaired sexual function and erectile dysfunction. Flucytosine is a water-soluble, fluorinated pyrimidine that possesses excellent bioavailability. It is administered only in combination with amphotericin B because of frequent development of secondary drug resistance, and is associated with dose-dependent bone marrow suppression.

According to a study, the most frequent side effects were nausea and vomitus, hypertriglyceridemia, hypokaliemia, increased liver enzyme levels indicating liver toxicity and skin reactions. At least one of these side effects was observed in 39% of patients systemically treated with azoles for >4 weeks. These side effects may require dose reductions and thus result in a loss of efficacy and in some cases even in discontinuation of treatment (Bennett JE: Antimicrobial Agents: Antifungal Agents. In: Goodman & Gilman's The Pharmacological Basis of Therapeutics (JG Hardman & LE Limbird, eds), McGraw-Hill, New York 10: pp 1175-1190, 1995).

In addition to these direct toxic effects, all azole derivatives are afflicted with severe drug-drug interactions. This is based on the mechanism of action of these drugs. The target enzyme inhibited in the fungus is closely related to the main mammalian drug metabolising liver enzyme, the cytochrome P450 type 3A4 (CYP3A4). All azoles including ketoconazole, itraconazole and other related compounds are potent P450 enzyme inhibitors with a special focus on CYP3A4. This results in strong drug-drug interactions. In transplantation patients or other patients receiving cyclosporin for example, the plasma level of cyclosporin is largely increased as a result of azole induced CYP inhibition. Some authors even propose to use these drugs to reduce the dose of cyclosporin needed. A combination of several drugs with these enzyme inhibitors is problematic and even contraindicated. Midazolam, triazolam, chinidin, pimozid, dofetilid as well as β-HMG-CoA reductase inhibitors are contra-indicated in combination with azole derivatives, to name only a few. Due to the effect on plasma electrolytes, namely on potassium, azoles may not be administered in patients with heart failure.

As can be seen from this list which is not complete, the treatment of patients with ketoconazole, itraconazole and other azole derivatives is by no means easy and without risks. The situation is further complicated in severely ill patients. As indicated above, organ mycoses are especially a problem in severely ill patients. Therefore, despite the availability of different potent antifungal drugs, the treatment of organ mycoses remains a big problem. Even if successful treatment may be reached by increasing the dose, the side effect profile calls for low doses to be administered to reduce the systemic availability resulting in toxicity.

A theoretical approach to reduce side effects is the selection of different routes of administration to increase local drug concentration while reducing systemic drug exposure. The most widely investigated agent utilising alternative methods of drug delivery is amphotericin B. Dosage forms for this agent include topical dosage forms (aerosol, nasal spray, irrigations, pastes, absorbable sponges, impregnated bone cement and gelatin), oral dosage forms (solutions, suspensions, tablets and so on) and ophthalmic dosage forms (drops, ointments and injections). Amphotericin B has been administered by routes such as oral, endobronchial, intrathecal, intracisternal, intra-articular, intraperitoneal, ophthalmic and as an antibiotic 'line lock'. Also, nystatin has been administered as an aerosol, percutaneous paste and bladder washes.

Azole derivatives, however, such as miconazole, fluconazole, ketoconazole and posaconazole, have been administered by alternative methods to a lesser degree. This is based on the physicochemical properties of the azoles. While amphotericin B is water soluble and can be administered as aerosol as is also the case for nystatin, it had been assumed that administration of azoles, such as miconazole, fluconazole, ketoconazole, voriconazole and posaconazole as aerosol is not effective, since these compounds are highly water insoluble preventing, the formulation of a solution to be administered as aerosol, even with the help of solubilising agents.

Alternative formulations which involve the generation of microcrystalline or nanocrystalline suspensions are described in the literature, but none has reached the market or is undergoing active development.

For inhaled administration, special caution is to be taken with regard to composition of any formulation. This is because the lung has very limited capacity and capability to clear insoluble particles after inhalation. Such lack of capability to clear insoluble (anorganic) particles can lead to severe problems, especially in case of long term exposure. Such problems include cancer induction or cancer promotion as described, for example, for asbestos particles or diesel exhaust (Valberg PA, Watson AY. Lack of concordance between reported lung-cancer risk levels and occupation-specific diesel-exhaust exposure. Inhal Toxicol. 2000;12(suppl 1):199-208) but also for other insoluble particles as shown in numerous scientific papers. Alternatively, chronic lung inflammation and tissue destruction is described. An example of such destructive effects is the silicate lung found in employees exposed to high loads of silicate dust.

Recently, the unexpected high relevance and toxicity of nanocrystalline insoluble particles has been described and characterized, see for review Oberdörster G, Oberdörster E, Oberdörster J. Nanotoxicology: an emerging discipline evolving from studies of ultrafine particles. Environ Health Perspect. 2005;113:823-839. The authors describe that, when inhaled, specific sizes of nanoparticles are efficiently deposited by diffusional mechanisms and phagocytosis in all regions of the respiratory tract. The small size facilitates uptake into cells and transcytosis across epithelial and endothelial cells into the blood and lymph circulation to reach potentially sensitive target sites such as bone marrow, lymph nodes, spleen, and heart.

Access to the central nervous system and ganglia via translocation along axons and dendrites of neurons has also been observed. These findings affecting the whole body are seen in addition to the direct effects of insoluble nanoparticles on lung tissue where a first reaction is of inflammatory nature. This background of information has to be taken in consideration if microcrystalline or nanocrystalline formulations are to be judged whether they are indeed adequate to be used for chronic inhalation or whether they pose a relevant health risk.

If insoluble particles are to be administered via inhalation, the aims of such a formulation consisting of crystals of different size in a formulation can be defined as follows:

A formulation must consist of adequate particles or droplets, which can be inhaled and deposited in the lung. According to common knowledge, the best lung deposition is reached with particles or droplets having a mass median aerodynamic diameter of 1 to 5 µm. Furthermore, the generation of small crystals is designated to increase the surface of the compound to enable dissolution of the active compound. It is common sense that only dissolved compounds can reach the biological target in the tissue and can be biologically active. Finally, the formulation selected must be easy to handle and stable and devices need to be available which can be used to administer these formulations at adequate doses sufficient to reach a high tissue level within an acceptable time frame. Despite the knowledge of special toxicity of nanoparticulate, insoluble (anorganic) particles, to date no reference is made to qualify the production procedure and the resulting formulation with regard to potential particulate insoluble contamination. However, such contamination is highly expected, especially if milling processes are used to achieve the intended particle size of active compound.

Wet milling, for example, is used as a method to achieve homogenous nanosuspensions with adequate particle size distribution. To establish a good grinding success, beads with a very hard surface are commonly used.

Adequate materials may be steel, glass, or zirconium oxide, to name a few. Despite the high density surface, such milling beads have a high wear upon usage, i.e. during the grinding process not only the active ingredient is comminuted, but (to a lesser extent) also the grinding beads are partially abraded. This results in a substantial content of grinding beads' wear in any suspension generated with such beads. According to the specification of, for example, zirconium oxide beads, the wear can be as large as 3-6% of the bead material for one grinding process. For steal and glass beads, the percentage can be even higher, reaching up to 10% of the grinding bead weight, depending on the grinding time required. On average, abrasion can be in the range of 3-10% of grinding bead weight, depending on the material used and the grinding times. Other processes useful to produce adequate nanoparticles are afflicted with similar risks for contamination with particulate insoluble matter. This results is a substantial health risk. While respective nanosuspensions may well be orally ingested with limited risk, the inhalation has to be judged differently due to the low clearance of insoluble inorganic particles from the lungs.

Other procedures such as high pressure homogenization are not capable of producing highly concentrated nanosuspensions as are required for high dose treatment of lung mycosis, since these procedures require low viscosity of the starting suspension which is typically achieved with low solid matter content in the order of 5% (w/w) of the total volume or even lower. Further more, these methods are also afflicted with surface abrasion. Actually, the highest possible concentration of active compound to be reached with high pressure homogenization is in the range of about 5%, preventing the administration of sufficient doses to patients afflicted with lung infections. Considering that a dose of about 100 mg per administration of antifungal medication is to be deposited in the lung and further considering that the maximal deposition of inhaled drug substance is in the range of 30% of the nebulised drug substance (the reminder is swallowed or exhaled), the time for administration can be easily calculated. Standard nebulisers such as "Pariboy" are capable of nebulising 3 ml fluid within 20 min. An acceptable inhalation time which a patient can follow daily or even two to four times per day may be in the range of 15 to 20 min. Based on these figures, we can calculate that using a 10% drug suspension 300 mg may be nebulised within 20 min and 90 mg can be expected to be deposited in the lung. Using a 5% suspension, the time would have to be doubled which is hardly acceptable for a daily treatment. This easy calculation indicates that 10% or even higher concentrated suspensions are more suitable for oral inhalation as compared to low concentration (5% and below) formulations.

WO 2004/060903 A2 describes a method for treating and/or providing prophylaxis against pulmonary fungal infections, comprising delivering an aerosolized pharmaceutical formulation comprising an antifungal agent to the lungs. The antifungal agent, e.g. an azole derivative, is employed in micronized form, the particles being less than 3 µm in diameter. The particles may be obtained using suitable size reduction processes, such as supercritical fluid processing methods, cryogenic milling, wet milling, ultrasound, high pressure homogenization, microfluidization and crystallization processes. However, WO 2004/060903 A2 does not refer to a safe production and use of nanosuspensions and especially does not deal with inorganic insoluble contaminations. No safety data are shown to display the safety and tolerability of the formulations obtained.

DE 101 45 361 A1 describes a method for preparing a liquid and sterile preparation for inhalation, the method comprising preparing an aqueous suspension of an active agent hardly soluble in water, reducing the particle size of the active agent and performing heat sterilization. Reduction in particle size is carried out using high pressure homogenization, the latter resulting in particle sizes in the range of from 500 nm to 2 µm. However, this particle size is suboptimal for inhalation of poorly soluble active agents, such as azole derivatives, since such particles show rapid sedimentation and lead to less nebulization efficiency as compared to true nanoparticulate formulations as described herein. In addition, faster drug dissolution is obtained by further reducing the size of the particles below 500 nm. Using nanoparticles most fluid droplets generated for inhalation with a diameter of 2-5 nm or below will contain also active material, while using larger particles only a smaller fraction of droplets can contain compound. This is due to the fact that for a 10% suspension the average droplet contains 10% active matter and therefore must contain 90% fluid. Furthermore, the safety and tolerability of this formulation is not shown.

US 2005/0048126 A1 describes a composition of an aqueous suspension of submicron- to micron-size particles of an antimicrobial agent that renders the agent potent against organisms normally considered to be resistant to the agent. In detail, the antimicrobial agent may be an azole derivative which is administered with a particle size in the range of from about 150 nm to about 1 µm, which particle size may be obtained using high pressure homogenization or mechanical grinding, such as jet milling, pearl milling, ball milling, hammer milling, fluid energy milling or wet grinding techniques. The preferred detergent was determined to be poloxamer 188 which was added at a concentration of 10% as compared to the active ingredient. Again, no hints for *in vivo* tolerability or safety are given.

Based on this overview, it becomes clear that while a number of different options for crystalline formulations of azole derivatives are described aiming at local treatment of fungal infections within the respiratory tract, none of these formulations is optimal for inhaled administration, being either not sufficiently pure regarding inorganic contaminations or being not sufficiently milled to adequate diameters or are not sufficiently milled to sizes below 400 nm or are not sufficiently concentrated to a concentration in the range of 10% or above.

### Detailed Description of the Invention

In view of the above, there still is a need for a safe and highly concentrated formulation which can be used for inhalation to administer antifungal medication with low solubility. Such formulation must not only be effective, but especially safe and almost completely free of insoluble inorganic particulate contaminations.

Thus, it was an object of the present invention to provide a pharmaceutical composition which at least partially overcomes the above deficiencies. More particularly, such pharmaceutical composition should fulfil the following goals:
- High concentration (10% or above) to enable short inhalation times for high dose administration
- High respiratory tract deposition to increase pharmacological (prophylactic or therapeutic) effect
- Lung retention to reduce systemic exposure and to increase duration of action in the lung
- Rapid metabolism after systemic absorption to reduce systemic exposure resulting in fewer systemic side effects
- In addition, a formulation should be able to not only reach the oropharyngeal area and lung tissue, but also the adjacent lung lymphoid tissue to eliminate fungi which had been phagocytosed and transported to the lymphoid tissue.
- The formulation should be highly safe and must show excellent local tolerability, especially for long term use and repeated dose administration.
- Since the formulation is to be inhaled, no inorganic residues and especially no nanocrystalline particles in the form of inorganic insoluble residues, possibly derived from the milling process or the homogenization process, are acceptable.

This object was achieved according to the present invention by providing a pharmaceutical composition for administration by inhalation, comprising as an active agent at least one antifungal azole derivative and optionally pharmaceutically acceptable diluents and/or adjuvants, characterized in that the pharmaceutical composition has an inorganic particulate impurity of < 10000 µg/l and is obtainable by a method comprising the steps:
a) providing at least one antifungal azole derivative and optionally pharmaceutically acceptable diluents and/or adjuvants,
b) wet milling the at least one antifungal azole derivative and the optionally pharmaceutically acceptable diluents and/or adjuvants using a pearl mill and smooth milling beads composed of at least one organic material.

According to the present invention, the antifungal azole derivative may be in the form of a nanocrystalline powder, e.g. a nanocrystalline dry powder, or in the form of a nanosuspension of crystalline or amorphous particles. In case of a nanosuspension, the particles may be also completely or in part amorphous, as a result of the preparation of the nanosuspension.

In a preferred embodiment, the antifungal azole derivative is present in the pharmaceutical composition in the form of particles having an average particle diameter in the range of from about 50 nm to about 400 nm, preferably in the range of from about 100 nm to about 300 nm, and most preferably in the range of from about 150 nm to about 250 nm. Preferably, the particles of the antifungal azole derivative moreover show a narrow size distribution, as indicated by the polydispersity index (PDI). The PDI describes the size distribution of nanoparticles. The theoretical value is between 0 (each particle of the same size) and 1 (maximal deviation from homogeneous particle distribution). A low polydispersity index of nanoparticles warrants a targeted, reproducible and controlled lung distribution and an optimal compound release as well as uptake into lung cells, inflammatory cells and fungal cells.

Thus, in a preferred embodiment of the present invention, the antifungal azole derivative is present in the pharmaceutical composition in the form of particles having a polydispersity index of ≤ 0.3, preferably of ≤ 0.25, and most preferably of about 0.2.

The inhalation of nanoparticles, e.g. crystalline powders or suspensions, e.g. crystalline suspensions or amorphous and/or crystalline nanosuspensions, of azole derivatives results in a high pharyngeal and lung tissue exposure. Therefore, administration of azole derivatives by inhalation is efficacious and, compared to the systemic or oral administration, highly advantageous.

Unexpectedly, we found that, possibly due to the low solubility of the compounds and the nanoparticulate size below 400 nm, the crystals and/or amorphous nanoparticles of the antifungal azole derivative form a depot in the lung and are slowly released into the lung tissue. The compounds are also retained in the lung tissue after solubilisation due to their high protein binding characteristics. Both effects result in a lung retention. Furthermore, due to the nature of the drugs, the particles administered are incorporated into immune cells by phagocytosis and are transported to the lymph nodes where they are liberated resulting in a high exposure of the local lymph nodes. This dual effect, the exposure of the lung tissue including the tissue of the oropharyngeal area (oral cavity, nasal cavity, pharynx and larynx) and the lymph nodes, is important, since fungal hypes and fungal cells are phagocytosed and transported into the local lymph nodes where they can be released again.

The azole derivatives, if administered as crystalline or amorphous nanoparticles directly into the lungs via inhalation, fulfil the criteria for successful local treatment of fungal infections of the respiratory tract. While a high exposure of the oropharyngeal and respiratory tract can be assured, the absorption from the lung is slow reducing the systemic availability. This reduced systemic exposure is further reduced due to the rapid hepatic metabolism of the systemically available compound. The lung retention and the high tissue exposure in the lung further reduce the total dose to be administered as compared to oral or intravenous administration. The previously unknown property that the particles are incorporated into immune cells by phagocytosis and transported to the lymph nodes is especially interesting for the treatment of lung mycosis, since the fungi also invade the immune system. This mechanism may help to explain the superior pharmacological property of such suspensions.

In the pharmaceutical composition according to the invention, the antifungal azole derivative may be present in a concentration of about 10% (w/w) to about 30% (w/w), preferably in a concentration of from 10% (w/w) to 20% (w/w), and most preferably in a concentration of from 12% (w/w) to 18% (w/w), based on the total weight of the pharmaceutical composition.

The pharmaceutical composition is particularly suitable for the administration of azole derivatives, e.g. conazoles or N-acylmorpholine ring containing azole derivatives such as UR-9746 and UR-9751. Perferably, the antifungal azole derivative is selected from the group consisting of ketoconazole, itraconazole, fluconazole, voriconazole, posaconazole, clotrimazole, econazole, miconazole, oxiconazole, terconazole, tioconazole, sulconazole, ravuconazole, including pharmaceutically acceptable derivatives thereof, such as salts, esters or amides. In an especially preferred embodiment, the antifungal azole derivative is itraconazole.

The pharmaceutical composition may further comprise pharmaceutically acceptable carriers, diluents and/or adjuvants. Preferred pharmaceutically acceptable carriers are saccharides or sugar alcohols which may be selected e.g. from lactose monohydrate, anhydrous lactose, glucose, trehalose and/or mannitol. Preferred pharmaceutically acceptable diluents are water, aqueous buffer solutions, or mixtures thereof with a physiologically acceptable alcohol such as, for example, ethanol, propylene glycol and/or glycerol. Preferred pharmaceutically acceptable adjuvants are hydrophilic polymers, which may e.g. be selected from polyvinyl pyrrolidone, polyethylene glycol and/or polyvinylalcohol.

In a preferred embodiment, the composition comprises a surfactant, preferably a non-ionic surfactant, as a pharmaceutically acceptable adjuvant. Examples for surfactants are polyoxyethylene-polyoxypropylene copolymers, polysorbates, lecithins, phospholipids, polyethylene glycol esters or ethers of fatty acids and/or fatty alcohols. Preferably, the surfactant is a polysorbate, e.g. polysorbate 80, a poloxamer, e.g. poloxamer 188, Solutol^{®} HS15, or any combination thereof.

In a preferred embodiment, the present invention provides a pharmaceutical composition which comprises the at least one antifungal azole derivative and a surfactant in a weight ratio between 10:1 and 1:1. Preferably, the pharmaceutical composition comprises the antifungal azole derivative and the surfactant in a weight ratio between 10:1 and 10:2, and most preferably in a weight ratio of about 10:1.4.

For inhalative administration of azole derivatives, the following formulations and technologies are especially preferred:

### Dry powder formulation

The composition may be administered using standard dry powder inhalation technologies. For this purpose, a nanosuspension comprising the active agent and at least one surfactant in the above-mentioned ratio is spray dried to form a dry powder. The drying process preserves the particulate structure, i.e. the crystalline particle size distribution remains the same as in the suspension. This powder can be blended with an adequate carrier such as lactose or other carriers and excipients commonly used with dry powder inhalation formulations. The composition may be administered by usual dry powder inhalation devices, e.g. the well described Cyclohaler, Spinhaler or Rotahaler devices. However, other suitable devices may be used as well. The inhaled dose can be in the range of e.g. 10 mg per individual inhalation (Srichana T et al. Eur J Pharm Sci. 1998, 7:73-80).

Using dry powder inhalers is also an effective method to treat oropharyngeal fungal infections including oesophageal infections. Due to the nature of such devices, 10 to 30% of the active ingredient reaches the lung while 70 to 90% are deposited in the pharynx and are swallowed. This allows for an effective treatment of oropharyngeal infections including oesophageal infections. In addition, this treatment serves as a prophylactic treatment of the lung and the gastrointestinal system preventing the spread of the infection from the oropharyngeal site to the lung or the gastrointestinal system.

### Nanosuspension

In order to obtain a nanosuspension, crystalline and/or amorphous nanoparticles as described above are suspended in a suitable carrier medium, e.g. in an aqueous formulation containing suspension stabilizers and/or additives such as those mentioned above, to prevent the particles from sedimentation and to stabilize the suspension. The preparation of this pre-suspension is an integral part of the manufacturing procedure. This suspension contains 10 to up to 30%, preferably up to 20% of active compound, i.e. an azole derivative, and may be administered either using a nebuliser device which may utilize compressed air, ultrasound or a vibrating mesh to produce an aerosol with a median droplet diameter in the range of about 2 µm to about 5 µm. These droplets contain the nanocrystals. General technology to produce nanocrystal suspensions is well described in the literature. A common method to produce nanosuspensions of drugs is by the technology of high shear or high pressure homogenization, as disclosed in Jacobs, C., (Nanosuspensions for various applications, Free University of Berlin, Dissertation 2004, Department of Chemistry and Pharmacy, hftp://www.diss.fu-berlin.de/2004/329/indexe.htmi) and several other publications (see for example Moschwitzer et al., Development of an intravenously injectable chemically stable aqueous omeprazole formulation using nanosuspension technology. Eur J Pharm Biopharm. 2004;58:615-9).

To accomplish the aim of the present invention, high pressure homogenization was tested and applied to crystalline drug particles suspended in water and stabilized with one of the above mentioned surfactants and/or polymers. Nanosuspensions with average particle sizes of 400-600 nm were obtained when the suspension of the crystalline antimycotic drug is homogenized at pressures between 500 and 1500 bar, whereas several passes through the homogenizer had to be applied.

A possible formulation obtained by high pressure homogenization contains up to 5% (w/w) of a crystalline antimycotic agent, 1% (w/w) surfactant, e.g. polysorbate and water, and is passed 10 to 20 times through the high pressure homogenizer at a pressure of 1000 bar, as a result of which a particle size of about 400 nm, or, depending on the surfactant used, 400-600 nm with a narrow particle size distribution is obtained.

However, two significant disadvantages of using high pressure homogenizers for the production of nanosuspensions are pertinent. One is the limited applicability of this technology to high concentration suspensions, namely suspensions having a solid content of > 5% (w/w), or even more above 10% (w/w). Since the clinical dose to be administered via inhalation for an adolescent or adult patient is preferably in the range of 100 mg or higher to be administered 1 to 4 times a day, the concentration of active ingredient is required to be high and should well exceed 5% to reduce the volume to be inhaled which is proportional to the time the inhalation takes. The second disadvantage is that the particles obtained are still relatively large and do not reach a particle size below 400 nm.

In contrast, the pharmaceutical compositions according to the invention are prepared by providing at least one antifungal azole derivative and optionally pharmaceutically acceptable diluents and/or adjuvants, and subsequently wet milling the at least one antifungal azole derivative and the optionally pharmaceutically acceptable diluents and/or adjuvants using a pearl mill and smooth milling beads composed of at least one organic material. According to this method, it is not only possible to achieve average particle diameters significantly below 400 nm, but to also provide a composition comprising the antifungal azole derivative in a concentration of up to 30% or even more than 30% (w/w).

A pearl mill essentially consists of a milling chamber which is filled with indifferent grinding media, in particular milling beads made from glass, steel or zirconium oxide. The problem encountered with milling beads composed of inorganic material is the abrasion of inorganic particles (i.e. glass, steel or zirconium oxide particles). Such abrasion can not be accepted for inhaled formulations since such particles are known to cause chronic lung inflammation and may even result in lung cancer.

Unexpectedly, it was now found that milling beads composed of at least one organic material are particularly suitable for producing pharmaceutical compositions for administration by inhalation, which compositions are also safe for long term treatment. The organic material may be any organic material suitable in the method according to the invention, e.g. an organic polymer. Milling beads composed of organic polymers, and in particular polystyrene and polycarbonate, are preferred media since they have a smooth and flexible surface and therefore show very low abrasion. Furthermore, no insoluble inorganic abrasion can occur. This is important since abrasion can be in the range of 3-10% of grinding bead weight, depending on the material used and the grinding times. As a result, the pharmaceutical compositions according to the invention exhibit an inorganic particulate impurity of < 10000 µg/l, preferably of < 5000 µg/l, and most preferably of < 2500 µg/l.

The milling beads, typically having a size in the range of from 150-650 micrometer, are filled into the milling chamber and moved by means of the rotation of the milling chamber or, in case the milling chamber is a stationary one, by means of a stirring device. The antimycotic drug is added to the milling chamber after it has been dispersed into a medium, e.g. water, in the presence of a stabilizer as described above. After wet milling the mixture for about 60 minutes to about 360 minutes, preferably for about 120 minutes to about 240 minutes, it is obtained a homogeneous highly concentrated nanosuspension with narrow particle size distribution and without the otherwise common contamination of insoluble inorganic material derived from the milling beads' wear. Typically, the drug content in such nanosuspension can be as high as 20% (w/w) to 30% (w/w), or even higher. Surfactant concentrations, needed to stabilize the milled particles, typically range from 5 to 100% relative to the drug substance.

A typical formulation to achieve the aim of the present invention comprises 5-30% (w/w), preferably 10-25% (w/w), more preferably 20% (w/w) antimycotic (active) drug, about 5-100% (w/w), more preferably about 10-25% (w/w) surfactant (e.g. polysorbate 80) relative to the drug substance, in a medium such as water or physiological buffer, such suspension being milled in a pearl mill using polystyrene milling beads for 240 minutes and resulting in an average particle size of 150-250 nm with narrow size distribution. The milling time may be varied from 120 minutes to 360 minutes and good results were obtained already after 120 minutes while a further milling beyond 240 min did not further improve the particle size distribution. However, milling times are related to the equipment used and may be adapted to the equipment as needed to achieve the required suspension.

A similar typical formulation to achieve the aim of the present invention contains the same amount of active compound and water but instead of polysorbate 80 a comparable amount of poloxamer 188, relative to the active ingredient, preferably about 5% (w/w) to 100% (w/w) relative to the drug substance. While these typical surfactants can be used either solely or in combination, other suspension stabilizers such as hydrophilic polymers including but not limited to polyvinyl pyrollidone, polyethylene glycol or polyvinyl alcohol and other adjuvants and surfactants as mentioned above are also well suited. If combinations of surfactants such as poloxamer 188 and polysorbate 80 are used, the individual concentration of each surfactant can be further reduced without compromising stability, particle size, or particle size distribution of the nanosuspension. For combinations, the relative fraction of each surfactant can range from 10% (w/w) to 100% (w/w); relative to the other surfactant. The total combined surfactant content ranges from 5% (w/w) to 50% (w/w) relative to active drug substance, depending on the stability and re-suspension quality needed. While stability and homogeneity of the suspension are important, the prevention of crystal growth is an additional goal accomplished by the surfactants added. Crystal growth derives from the soluble fraction of the active ingredient. For Itraconazole, the lowest crystal growth could be found if polysorbate 80 was used alone or in combination with poloxamer 188.

While the above mentioned nanosuspensions were prepared using 20% (w/w) of active compound, in different batches the milling process was performed using suspensions containing also 5% (w/w) compound. In these experiments, the surfactant content was also proportionally reduced relative to the reduction of the active compound and was kept in the range of 10 to 100% (w/w) of active compound, i.e. 0.5% to 5% of total volume. This resulted in a similar particle size distribution. These experiments indicate that the process is highly stable and easily scalable while factors such as surfactant content and active drug content are to be selected to fit the final clinical needs.

The resulting nanosuspensions containing for example 20% of active azole compound can be diluted with demineralised water or with salt containing solutions such as physiological salt solution or hyperosmolar salt solutions to adapt osmolarity to the needs of lung applications. Indeed, salts may be added already during the milling process to achieve suspensions with osmolarity in the range of 280 to 360 mOsmol. The dilution ratio can be in the range of 1:0.5 to 1:10, i.e. for example to 2 ml of a 20% suspension 1 to 20 ml of solvent may be added. The solvent (pharmaceutically acceptable diluents) may contain in addition to the above mentioned salts preservatives, surfactants or other pharmaceutically active agents in solution which need to be administered as co-medication according to the medical needs of the patient.

The solvent or pharmaceutically acceptable diluent may not only be water or a salt solution to adjust the osmolarity. Likewise, the pH may be adjusted using pharmaceutically acceptable buffer systems such as phosphate buffer solutions, Tris buffers or similar buffers. The pH may be adjusted to be in the physiological range, i.e. 7.0 to 7.6, most preferably 7.4. The pH may as well be adjusted to be slightly acidic using citric acid or similar biologically acceptable weak acids to prevent bacterial growth. As with the salts, the buffer ingredients may be added before grinding and may be present throughout the milling process without disturbing the milling process. They may however be also added after the milling in a dilution step prior to inhalation.

While all above mentioned suspensions are water based, a pharmaceutically acceptable diluent being used either during the milling process or for a dilution step may be a mixture of water and a physiologically acceptable alcohol. The alcohol content may range from 0.1 % to 30% (v/v), preferably in the range of 3 to 10% (v/v).

The resulting nanosuspensions may be sterilized by heat (i.e. autoclave). Alternatively, the nanosuspensions may be produced using aseptic processing or may be sterilized by radiation. Due to the nature of the process, i.e. the intensive milling to particle sizes well below the average size of bacteria, pathological germs are already destroyed. To prevent bacterial growth, preserving agents may be added which are well known in the literature.

All resulting suspensions are homogenous and stable at room temperature. Depending on the content of surfactant and the content of active ingredient, a slow separation of an aqueous phase above the suspension can be observed within days to weeks. This separation does not affect the particle size distribution of the suspension and, upon stirring or shaking, the nanosuspension is easily re-dispersible without any impairment of the size distribution.

The nanosuspensions which production has been described here may then be aerosolized using common technology, namely by means of an ultrasonic nebuliser, a compressed-air nebuliser, a membrane nebuliser or any other device being capable to produce a soft mist aerosol out of the nanosuspension. In contrast to the nebulisation of a microsuspension which contains micronized particles in the size range of 2 to 10 microns, which by knowledge tend to leave a huge drug residue within the nebuliser, the nanosuspensions prepared as described in this application was found to deliver the medication from the nebuliser reservoir more efficiently, resulting in low drug retention in the reservoir and higher drug delivery to the patient. Furthermore, due to the smaller size of the particle, most aerosol droplets can be expected to carry drug particles while upon nebulisation of micronized particles the distribution of the drug is more inhomogeneous. Therefore, the nanosuspension is distributed more evenly and more deeply into the lung, leading to an overall higher lung deposition of the medication and thus a better pharmacological effect.

The dose to be administered to treat an overt infection of the respiratory tract is usually in the range of about 1 mg to about 1000 mg per day, preferably in the range of about 10 to about 400 mg/day. The dose may be administered as a single daily dose or as a plurality of up to e.g. 4 individual doses per day, or as continuous administration for patients being continuously treated such as intensive care patients. For preventing infections or for maintaining therapy to prevent the exacerbation after successful treatment of an acute infection, the dose needed is lower and preferably in the range of 5 mg to about 100 mg/day. The above mentioned doses are calculated to fit the needs of an average adolescent or adult human being. Dependent on the body weight or the body surface, the dose may be individually modified to fit the needs of the individual patient. Other factors, including but not limited to factors such as disease stage, location of the infection in the lung, individual susceptibility, fungus strain and resistance may also have effect on the individual dose to be administered to the patient.

The pharmaceutical composition is suitable for use in human or veterinary medicine. Preferably, the patient is a mammal, including but not limited to human being, or a non-mammal animal such as a bird or a reptile such as a snake, in need of administration of an antifungal therapy, particularly to the respiratory tract. The duration of treatment may be single dose, or multiple administration over prolonged periods. In special circumstances, treatment may take part for the whole life span, either daily or in individual treatment courses separated by drug free periods.

The pharmaceutical composition is particularly suitable for the prevention and/or treatment of fungal infections, preferably of fungal infections of the respiratory tract including the lung and optionally adjacent lymphoid tissues. In particular, the pharmaceutical composition is suitable for the prevention and/or treatment of fungal infections in immune-compromised or immune-suppressed patients, such as HIV-patients, organ transplantation patients, cancer patients, newborns or patients under corticosteroid treatment.

The drug administration can be prophylactic in patients at risk such as organ transplantation patients or HIV-patients, patients with cystic fibrosis or patients under intensive care with a compromised or suppressed immune system or in patients which have been successfully treated for fungal respiratory tract infections to prevent re-infection. A different application is the administration to patients exposed to a high aerosol load of fungal spores to prevent development of a disease. Such treatment in the case of exposure or suspected exposure without immanent symptoms is called metaphylaxis.

The drug administration is therapeutic if symptoms of the disease are already evident or if the infection is detected or suspected. In all cases, the inhalation of azole derivatives is feasible and advantageous compared to the oral or systemic (for example intravenous) administration.

The inhalation of azole derivatives is particularly suitable for the prevention and/or treatment of fungal infections by Aspergillus species, Histoplasma species, Candida species, Pneumocystis species, Scedosporium species, Cunninghamella species Cryptococcus species, Coccidioidomyces species, Blastomyces species, Paracoccidioidomyces species, Sporothrix species, Onychomyces species, Dermatophyton species, Pseudallescheria species, Penicillium species and/or Scopulariopsis candida and/or other pathogenic fungi.

The inhalation of azole derivatives is e.g. useful for the prevention and treatment of aspergillosis, histoplasmosis, candidosis, cryptococcosis, coccidioidomycosis, blastomycosis, paracoccidioidomycosis, (for example *Paracoccidioides brasiliensis),* sporotrichosis, (for example *Sporothrix schenckii),* onychomycosis, and dermatophytoses. Other potentially pathogenic fungi to be treated include but are not limited to *Pseudallescheria boydii,* penicillium species such as *Penicillium marneffei* and *Scopulariopsis candida.*

The pharmaceutical composition may be administered as monotherapy or as combination therapy with a further active ingredient. The active ingredient may be a further antifungal agent, a local antiseptic, an antibiotic, an anti-inflammatory agent including corticosteroids, an agent to treat cystic fibrosis, a mucolyic, a bronchodilating agent, and any combination thereof. These active ingredients may be co-administered via inhalation or may be administered via alternative routes (oral or parenteral) in addition to the inhaled administration of antifungal medication.

Further anti-fungal agents include but are not limited to amphotericin B, flucytosine, caspofungin, terbinafine, naftifine, and griseofulvin. The combination of fluoroquinolone antibiotics, such as trovafloxacin or ciprofloxacin, with azole derivatives appears to enhance the antifungal activity of azole derivatives and is, therefore, also a preferred antifungal combination.

Local (oral) antiseptics include benzoic acid, chlorhexidine, triclosan, thymol, eucalyptol, methyl salicylate, alcohol. Antibiotics include but are not limited to antibiotics used to treat infections in cystic fibrosis such as macrolide antibiotics, ciprofloxacin, ofloxacin tobramycin including TOBI^{®}, an aerosol antibiotic used to treat lung infections, gentamicin, azithromycin, ceftazidime, cephalexin, cefaclor, piperacillin, imipenem or the new dry powder inhaled antibiotic colistin. Anti-inflammatory agents include but are not limited to corticosteroids (preferably inhaled steroids) such as budesonide and fluticasone and inhibitors of the phosphodiesterase enzyme 4 such as roflumilast. Agents to treat cystic fibrosis include but are not limited to mucolyics such as human DNAse and N-acetylcysteine, bronchodilators such as β2-receptor-agonists such as salbutamol or anticholinergics such as ipratropium bromide or theophylline, a mixed adenosine antagonist/phosphodiesterase inhibitor. A combination of all the above mentioned agents may be successfully co-administered or combined with inhaled azole derivatives.

In summary, the features described above result in a pharmaceutical composition exhibiting the following unexpected advantages:
- Higher success rate to treat fungal infections of the respiratory tract
- Reduced systemic side effects due to high local exposure and reduced systemic exposure
- No chronic toxicity stemming from inorganic insoluble particulate contamination
- Lower systemic exposure resulting in reduced drug interaction potential

Further, the present invention shall be explained in more detail by the following examples.

### Examples

### Example 1: Preparation of nanosuspensions using wet milling

All suspensions were milled in a pearl mill with a horizontal milling chamber which was cooled down to 10°C during the milling process so that the resulting temperature of the suspension was lower than room temperature. The milling chamber was filled with 80% of grinding media. The suspension was circulated by a peristaltic pump during the process.

The particle size was measured by laser light scattering. The Z average is the intensity weighted mean hydrodynamic size of the ensemble collection of particles measured by dynamic light scattering and is given in nanometers (nm).

Respective milling devices are produced by several companies and are produced for different batch sizes ranging from small volumes below 100 ml to batch sizes well above 100 I. For the examples given below, a mill manufactured by VMA Getzmann, Reichshof, Germany, but modified to be fed with a peristaltic pump was utilized. As milling media, several different milling media were tried but most experiments were performed with polymer milling media. The used polymers were:

### Polystyrene:

Polystyrene is a very tough and wear resistant non-toxic polymer of a very low density. The beads were of spherical design, clean and free of dust.. The grinding beads typically had a size in the range of 150-900 micrometer.

### Polycarbonate:

The polycarbonate beads were of cylindrical design with high density. It is a non toxic polymer which is non abrasive, clean and dust free. The cylindrical beads had a diameter of 300-400 micrometer and a length of 500-700 micrometer.

The used zirconium oxide grinding beads had a spherical design and a size of 300-700 micrometer.

All suspensions obtained could be easily diluted with water or other diluents, such as physiological sodium chloride solution, phosphate buffer solutions, ethanol containing aqueous solutions and the like. Using a higher solid payload in the milling chamber did not change the typical particle size distribution obtained.

Examples 1a-e describe the preparation of an Itraconazole nanosuspension using polysorbate 80 as surfactant.

### Example 1a

1.25 g of polysorbate 80 was dissolved in 93.75 g of double distilled water. 5 g of Itraconazole were added to this solution and dispersed with an Ultra-Turrax at room temperature. The suspension was then filled into the milling chamber filled with polystyrene grinding media and was milled for 4 hours. The resulting nanosuspension showed a particle size of 180 nm. The suspension remained stable over a period of several months.

### Example 1b

20 g of polysorbate 80 was dissolved in 60 g of double distilled water. 20 g of Itraconazole were added to this solution and dispersed with an Ultra-Turrax at room temperature. The suspension was then filled into the milling chamber filled with polystyrene grinding media and was milled for 4 hours. The resulting nanosuspension showed a particle size of 162 nm. The suspension remained stable over a period of several months.

### Example 1 c

1 g of polysorbate 80 and 2.5 g of polyethylene glycol 600 were dissolved in 91.5 g of double distilled water. 5 g of Itraconazole were added to this solution and dispersed with an Ultra-Turrax at room temperature. The suspension was then filled into the milling chamber filled with polystyrene grinding media and was milled for 4 hours. The resulting nanosuspension showed a particle size of 217 nm. The suspension remained stable over a period of several months.

### Example 1 d

5 g of polysorbate 80 was dissolved in 90 g of double distilled water. 5 g of Itraconazole were added to this solution and dispersed with an Ultra-Turrax at room temperature. The suspension was then filled into the milling chamber filled with polycarbonate grinding media and was milled for 4 hours. The resulting nanosuspension showed a particle size of 229 nm. The suspension remained stable over a period of several months.

Effect of the grinding time on the particle size for example 1d:

| grinding time | Z average [d.nm] |
|---|---|
| 30 minutes | 469 |
| 60 minutes | 350 |
| 120 minutes | 262 |
| 180 minutes | 242 |
| 240 minutes | 229 |

### Example 1e

1.25 g of polysorbate 80 was dissolved in 93.75 g of double distilled water. 5 g of Itraconazole were added to this solution and dispersed with an Ultra-Turrax at room temperature. The suspension was then filled into the milling chamber filled with circonium oxide grinding media and was milled for 4 hours. The resulting nanosuspension showed a particle size of 190 nm. The suspension remained stable over a period of several months.

### Example 1f

1.4 g of polysorbate 80 was dissolved in 88.6 g of double distilled water. 10 g of Itraconazole were added to this solution and dispersed with an Ultra-Turrax at room temperature. The suspension was then filled into the milling chamber filled with polystyrene grinding media and was milled for 4 hours. The resulting nanosuspension showed a particle size of 171.5 nm. The suspension remained stable over a period of several months.

### Example 1 g

2.8 g of polysorbate 80 was dissolved in 77.2 g of double distilled water. 20 g of Itraconazole were added to this solution and dispersed with an Ultra-Turrax at room temperature. The suspension was then filled into the milling chamber filled with polystyrene grinding media and was milled for 4 hours. The resulting nanosuspension showed a particle size of 169.4 nm. The suspension remained stable over a period of several months.

Summary of prepared nanosuspensions of examples 1 a-1 g

| | **grinding media** | **active ingredient** [**%]** | **excipient polysorbate 80 [%, relative to active ingredient]** | **Z-Average d.nm** |
|---|---|---|---|---|
| Example 1a | polystyrene | 5 | 25 | 180 |
| Example 1b | polystyrene | 20 | 100 | 162 |
| Example 1c | polystyrene | 5 | 20(in addition with 50% PEG 600) | 217 |
| Example 1 d | polycarbonate | 5 | 20 | 229 |
| Example 1 e | zirconium oxide | 5 | 25 | 190 |
| Example 1f | polystyrene | 10 | 14 | 171.5 |
| Example 1g | polystyrene | 20 | 14 | 169.4 |

### Examples 1 h-i describe the preparation of an itraconazole nanosuspension using poloxamer 188 as surfactant.

### Example 1h

5 g of poloxamer 188 was dissolved in 90 g of double distilled water. 5 g of Itraconazole were added to this solution and dispersed with an Ultra-Turrax at room temperature. The suspension was then filled into the milling chamber filled with polystyrene grinding media and was milled for 4 hours. The resulting nanosuspension showed a particle size of 207 nm. The suspension remained stable over a period of several months.

### Example 1i

20 g of poloxamer 188 was dissolved in 60 g of double distilled water. 20 g of Itraconazole were added to this solution and dispersed with an Ultra-Turrax at room temperature. The suspension was then filled into the milling chamber filled with polystyrene grinding media and was milled for 4 hours.

The resulting nanosuspension showed a particle size of 212 nm. The suspension remained stable over a period of several months.

Summary of prepared nanosuspensions of examples 1h-1i

| | **grinding media** | **active ingredient [%]** | **excipient poloxamer 188 [%, relative to active ingredient]** | **Z-Average [d.nm]** |
|---|---|---|---|---|
| Example 1h | polystyrene | 5 | 100 | 207 |
| Example 1i | polystyrene | 20 | 100 | 212 |

Examples 1j-k describe the preparation of an itraconazole nanosuspension using Solutol^{®} HS15 as surfactant.

### Example 1j

5 g of Solutol^{®} HS15 was dissolved in 95 g of double distilled water. 5 g of Itraconazole were added to this solution and dispersed with an Ultra-Turrax at room temperature. The suspension was then filled into the milling chamber filled with polystyrene grinding media and was milled for 4 hours. The resulting nanosuspension showed a particle size of 201 nm. The suspension remained stable over a period of several months.

### Example 1 k

20g of Solutol^{®} HS15 was dissolved in 60g of double distilled water. 20g of Itraconazole were added to this solution and dispersed with an Ultra-Turrax at room temperature. The suspension was then filled into the milling chamber filled with polystyrene grinding media and was milled for 4 hours. The resulting nanosuspension showed a particle size of 153 nm. The suspension remained stable over a period of several months.

Summary of prepared nanosuspensions of examples 1j-1k

| | **grinding media** | **active ingredient [%]** | **excipient Solutol^{®} HS15 [%, relative to active ingredient]** | **Z-Average [d.nm]** |
|---|---|---|---|---|
| Example 1j | polystyrene | 5 | 100 | 201 |
| Example 1k | polystyrene | 20 | 100 | 153 |

### Example 2: Determination of inorganic impurity in a nanosuspension generated using wet milling with polystyrene milling beads in comparison to a nanosuspension obtained by wet milling with zirconium oxide milling beads stabilized with cerium

The wear rate of the grinding media of a nanosuspension produced by wet milling was determined by "Inductively Coupled Plasma" (ICP) analysis. ICP is an analytical method for trace analysis of elements.

The suspensions were prepared as usual, milled for 4 hours, and were subsequently analysed. Table 1 shows the determined wear rate in a suspension milled with polystyrene beads and with zirconium oxide beads stabilized with cerium. According to the elements of the grinding media and the parts of the mill, the analysis was carried out for the elements iron, cerium and zirconium.

**Table 1: Wear rate of polystyrene beads and zirconium oxide beads**

| | **polystyrene beads [mg/L]** | **zirconium oxide beads [mg/L]** |
|---|---|---|
| iron | 0.74 | 1.9 |
| cerium | 0 | 12 |
| zirconium | 0.58 | 110 |

### Example 3: Effect of the milling process on the reduction of contaminating organsims

To evaluate the effect of the milling process on the reduction of contaminating organisms, a suspension spiked with a predetermined level of contaminating organisms was milled for four hours.

The suspension was prepared as usual, and one millilitre of a suspension of Brevindumonas diminuta was added, resulting in an initial microbial number of 22533 colony forming units (cfu)/ml in the suspension. The bacterial count was determined before and after pre-suspending the suspension with an Ultra-turrax. Then the suspension was given into the milling chamber and the process was started. Samples were taken after 30, 60, 120, 180 and 240 minutes.

**Table 2: Bacterial count over process time**

| | **cfu / ml** |
|---|---|
| before Ultra-turrax | 6250 |
| after Ultra-turrax | 4900 |
| | |
| **30** min | 1345 |
| **60** min | 625 |
| **120** min | 200 |
| **180** min | 225 |
| **240** min | 400 |

### Example 4: Lung deposition and lung kinetics of Itraconazole

For this experiment, male Wistar rats are utilized. Itraconazole is administered as dry powder formulation by direct intratracheal administration to mimic the lung deposition fraction of a dry powder inhalation. The following technology is used for drug administration: Micronized crystalline itraconazole (particle size about 1-5 µm) is blended with lactose (respitose 325M^{®}, DMV technologies, Netherlands) at a rate of 1:1. A total volume of 20 mg/kg, i.e. 10 mg/kg itraconazole is filled into a lockable one way stop cock which is connected to a small plastic tube on one side and a 5 ml syringe at the other side. The small plastic tubing is inserted under anaesthesia into the trachea and during the inspiration phase the dry powder is pushed into the lung using a volume of 2 ml air. This procedure assures a good distribution throughout the lung resembling dry powder inhalator device deposition. The tip of the tubing is positioned just before the bifurcation of the trachea.

After administration, the tubing is retracted and the rats are allowed to awake. At 6 different time points after administration starting 30 min after administration the lungs are harvested after killing the rats. Lung tissue is homogenized and drug concentration is determined using standard analytical methods. At each time point two rats are sampled.

A different group of rats is treated with a nanosuspension containing 10% of crystalline itraconazole (particle size about 100-200 nm) and 1.4% of polysorbate 80 (see formulation described in example 1f). For this purpose, a device from Pen Century, UK is utilized which is inserted into the trachea to the anesthetized rats. The suspension is sprayed into the trachea during the inspiration phase of the rat.

After 6 different time intervals following administration, groups of 3 rats each were sacrificed using an overdose of chloral hydrate, and the lung tissue is removed. The tissue content of itraconazole is analyzed using standard procedures. The tissue content at each time point is analyzed and the values of 3 rats are averaged. The terminal half life is calculated from the last 3 time points.

The data indicate that tissue concentrations range from 7-20 µg per g lung tissue (peak levels). The nanosuspension results in higher peak levels as compared to the dry powder administration. The compound is cleared from the lungs with a half life of approximately 5.2 +/- 1.9 hours for the nanosuspension and approximately 7 hours for the dry powder formulation indicating lung retention of both formulations.

### Example 5: Therapeutic effect of Itraconazole administered by inhalation in immune comprised NMRI mice

For this experiment, a method described by Liebmann et al. was used (Liebmann et al., 2004: Deletion of the Aspergillus fumigatus lysine biosynthesis gene lysF encoding homoaconitase leads to attenuated virulence in a low-dose mouse infection model of invasive aspergillosis. Archives of Microbiology 181, 378-383).

In brief, male Sprague-Dawley rats weighing 140 to 150 g received cortisone acetate (100 mg/kg, subcutaneously, thrice weekly), tetracycline via drinking water, and a low-protein diet. After 2 weeks on this regimen, animals were infected via the trachea with a suspension of 106 conidia of A. fumigatus H11-20 in 0.1 ml of sterile saline. In every trial, each treatment or control group consisted of 8 or 10 animals. Most trials included a total of six groups. The outcome was judged by survival analysis and histopathologic examination of the lungs. Tissues from animals at necropsy were homogenized in saline (9 ml/g), and slides were stained with toluidine blue-O. All animals were anesthetized with enflurane during all surgical procedures and dosing. Moribund animals were sacrificed with carbon dioxide gas. Survival analysis was done by Kaplan-Meier plots and the log rank test. A p-value of less than 0.05 was considered significant.

### Treatment regimens

For oral treatment, animals continued to receive the immunosuppressive regimen for 1 week postinfection and were treated twice daily with oral itraconazole at a dose of 10 mg/kg starting the day before inoculation or with placebo for 7 days.

For aerosol treatment, groups of four or five rats were placed in a Plexiglas chamber which was coupled to a "Pariboy" inhalation nebuliser which nebulises suspensions with pressurized air (4 I/min). The animals were allowed to breath the nebulised itraconazole suspension for 15 min twice daily (between 7 and 8 am and between 3 and 4 pm). The treatment was initiated 6 hours after the inoculation and was continued daily. Vehicle animals received the vehicle solution containing 1.4% polysorbate 80, while verum-treated animals were inhaled with a suspension of 10% itraconazole and 1.4% polysorbate 80 (taken from example 1f).

While in both vehicle control groups mice started to die on day 3 and while in the control group 11 of 12 rats had died by day 9, 8 out of 12 rats survived Until day 14, indicating a clear therapeutic effect of itraconazole administered by inhalation in the form of an aerosol nanosuspension. In the orally treated control group, 10 out of 12 mice had died by day 9, indicating that the treatment had failed in this group.

In a second experiment, male balb-c mice were utilized. The animals of all groups were immune-suppressed using 100 mg/kg body weight cyclophosphamide at day -4, -1, 0, 2, 5 and 8. On day -1, the animals received 100 mg/kg cyclophosphamide i.p. and 200 mg/kg cortisone acetate subcutaneous (24 ± 2 hours before infection). Additionally, the animals were treated by inhalation of itraconazole using the same inhalation device as described in the study above. At day 0, the animals were infected intranasally with 2.5 x 104 A. fumigatus conidia.

After the infection, the animals were observed for the following 14 days 3-5 times per day and the immune-suppressive treatment was continued on day 2, 5 and 8. Additionally, one group of mice used as reference group was treated with Amphotericin B i.p. using 3 mg/kg daily.

The drug effect was assessed as the number of surviving animals. As expected, 9 out of 12 control animals had already died on day 6 after infection and the remaining animals died with the exception of one until day 9. For animals treated as decribed above, i.e. using the 10% aerosol of itraconazole twice daily, a strong effect was seen. No animal had died by day 6 and only 3 out of 12 mice had died at the end of the observation period. This effect was superior to the effect observed for Amphothericin B, where the survival rate was good on day 6 with only 3 animals having died while at the end of the observation period 9 out of 12 mice treated with Amphothericin B had died.

### Example 6: Safety and tolerability of inhaled itraconazole

To evaluate the tolerability of inhaled itraconazole, rats (Wistar rats, 212-248 g) were exposed using an exposition chamber connected to a pressure driven inhalation device. For aerosol treatment, groups of two rats were placed in a Plexiglas tube (length: 28 cm, diameter: 14 cm) having a conical inlet and outlet part and a flat bottom, which tube was coupled to a "Pariboy" inhalation nebuliser which nebulises suspensions with pressurized air (4 l/min). To allow for a steady flow and even distribution, the nebulizer was connected to the chamber via a short tube (length: 10 cm, diameter: 22 mm), and the animal room in the chamber was separated from the inlet with a wall which was perforated with 84 small holes, each 3 mm diameter. By this way, the conical part of the inhalation chamber functioned as spacer. Similarly, the outlet was also separated with a similar spacer. This chamber design allowed for an even distribution of the nebulized compound within 25 sec after start of inhalation. The animals were allowed to breath the nebulised itraconazole suspension for 30 min once daily (between 8 and 10 am). On the 8^{th} day and 24 hours after the last dose, the rats were anaesthetized by an overdose of chloral hydrate. By opening of the abdominal aorta, the rats were exsanguined. The lungs were removed and filled with 4% buffered formaldehyde solution and prepared for histological examination by immersion in 4% formaldehyde. Histology was performed after HE stain. In addition, the animals were inspected for gross pathology. The following groups were tested: Group 1: air control; group 2: vehicle exposure, once daily 30 min for 7 days; group 3: itraconazole, once daily 30 min for 7 days. Vehicle animals received the vehicle solution containing 1.4% polysorbate 80 while verum-treated animals were inhaled with a suspension of 10% itraconazole and 1.4% polysorbate 80, taken from example 1f.

### Results

| | |
|---|---|
| Behaviour: | In all groups, no behavioural anomalities were observed. The compound treatment was well tolerated and all animals showed a similar rate of weight gain. |
| Pathology: | No findings were seen during section. |
| Histology: | All three groups of rats were inspected. No mucosal defects were observed. No compound desposits were found, neither in the lumen nor in the tissue. While mild to modest granulocyte and lymphotyte infiltration was observed, this finding was similar in all 3 dosing groups. |

In summary, the inhaled administration of itraconazle as nanosuspension (example 1f) was well tolerated. The content of polysorbate 80 used, i.e. 1.4% absolute or 14% relative to the content of itraconazole, had no influence on mucosal surface or lung tissue.

## Claims

1. A pharmaceutical composition for administration by inhalation, comprising as an active agent at least one antifungal azole derivative and optionally pharmaceutically acceptable diluents and/or adjuvants,
**characterized in that**
the pharmaceutical composition has an inorganic particulate impurity of < 10000 µg/l and is obtainable by a method comprising the steps:
a) providing at least one antifungal azole derivative and optionally pharmaceutically acceptable diluents and/or adjuvants, and
b) wet milling the at least one antifungal azole derivative and the optionally pharmaceutically acceptable diluents and/or adjuvants using a pearl mill and smooth milling beads composed of at least one organic material.

2. The pharmaceutical composition of claim 1,
**characterized in that**
the antifungal azole derivative is present in the form of particles having an average particle diameter in the range of from about 50 nm to about 400 nm, preferably in the range of from about 100 nm to about 300 nm, and most preferably in the range of from about 150 nm to about 250 nm.

3. The pharmaceutical composition of claim 1 or 2,
**characterized in that**
the antifungal azole derivative is present in the form of particles having a polydispersity index of ≤ 0.3, preferably of ≤ 0.25, and most preferably of about 0.2.

4. The pharmaceutical composition of any one of claims 1-3,
**characterized in that**
the antifungal azole derivative is present in a concentration of about 10% (w/w) to about 30% (w/w), preferably in a concentration of from 10% (w/w) to 20% (w/w), and most preferably in a concentration of from 12% (w/w) to 18% (w/w), based on the total weight of the pharmaceutical composition.

5. The pharmaceutical composition of any one of claims 1-4,
**characterized in that**
the antifungal azole derivative is selected from the group consisting of ketoconazole, itraconazole, fluconazole, voriconazole, eberconazole, posaconazole, clotrimazole, econazole, miconazole, oxiconazole, terconazole, tioconazole, sulconazole and ravuconazole.

6. The pharmaceutical composition of any one of claims 1-5,
**characterized in that**
the pharmaceutically acceptable diluent is water, or an aqueous buffer solution or a mixture thereof with a physiologically acceptable alcohol.

7. The pharmaceutical composition of claim 6,
**characterized in that**
wherein the physiologically acceptable alcohol is ethanol, propylene glycol and/or glycerol.

8. The pharmaceutical composition of any one of claims 1-7,
**characterized in that**
the pharmaceutically acceptable adjuvant is a hydrophilic polymer.

9. The pharmaceutical composition of claim 8,
**characterized in that**
the hydrophilic polymer is polyvinyl pyrrolidone, polyethylene glycol and/or polyvinyl alcohol.

10. The pharmaceutical composition of any one of claims 1-9,
**characterized in that**
wherein the pharmaceutically acceptable adjuvant is a surfactant.

11. The pharmaceutical composition of claim 10,
**characterized in that**
the surfactant is a non-ionic surfactant.

12. The pharmaceutical composition of claims 10 or 11,
**characterized in that**
the surfactant is a polyoxyethylene-polyoxypropylene copolymer, a polysorbate, a lecithin, a phospholipid or a polyethylene glycol ester or ether of a fatty acid and/or a fatty alcohol.

13. The pharmaceutical composition of any one of claims 10-12,
**characterized in that**
the surfactant is polysorbate 80, poloxamer 188, Solutol^{®} HS15, or any combination thereof.

14. The pharmaceutical composition of any one of claims 1-13,
**characterized in that**
it comprises the at least one antifungal azole derivative and a surfactant in a weight ratio of between 10:1 and 1:1, preferably in a weight ratio of between 10:1 and 10:2, and most preferably in a weight ratio of about 10:1.4.

15. The pharmaceutical composition of any one of claims 1-14,
**characterized in that**
the organic material is selected from the group consisting of polystyrene and polycarbonate.

16. The pharmaceutical composition of any one of claims 1-15,
**characterized in that**
the dose to be administered is in the range of about 1 mg to about 1000 mg per day, preferably about 1 mg to about 400 mg per day.

17. The pharmaceutical composition of any one of claims 1-16 for the prophylaxis, treatment and/or metaphylaxis of fungal infections, particularly of the respiratory tract including oral cavity, pharynx, larynx and/or lung.

18. The pharmaceutical composition of claim 17,
**characterized in that**
the fungal infection is an infection by Aspergillus species, Histoplasma species, Candida species, Pneumocystis species, Scedosporium species and/or Cunninghamella species, Cryptococcus species, Coccidioidomyces species, Blastomyces species, Paracoccidioidomyces species, Sporothrix species, Onychomyces species, Dermatophyton species, Pseudallescheria species, Penicillium species and/or Scopulariopsis candida.

19. The pharmaceutical composition of any one of claims 1-18 for use in human medicine.

20. The pharmaceutical composition of any one of claims 1-19 for the prophylaxis and/or treatment of fungal infections in immune-compromised or immune-suppressed patients.

21. The pharmaceutical composition of any one of claims 1-19 for use in HIV-patients.

22. The pharmaceutical composition of any one of claims 1-19 for use in organ transplantation patients.

23. The pharmaceutical composition of any one of claims 1-19 for use in cancer patients.

24. The pharmaceutical composition of any one of claims 1-19 for use in newborns.

25. The pharmaceutical composition of any one of claims 1-19 for use in patients suffering from cystic fibrosis.

26. The pharmaceutical composition of any one of claims 1-19 for use in patients under corticosteroid treatment.

27. The pharmaceutical composition of any one of claims 1-18 for use in veterinary medicine.

28. The pharmaceutical composition of any one of claims 1-27 for combination therapy with a further agent.

29. The pharmaceutical composition of any one of claims 1-28 for combination therapy with a further agent selected from a local antiseptic, an antibiotic, an anti-inflammatory agent including corticosteroids, an agent to treat cystic fibrosis, a mucolytic, a bronchodilating agent, a further antifugal agent and any combination thereof.

30. A method for preparing a pharmaceutical composition according to any one of claims 1-29, comprising the steps:
a) providing at least one antifungal azole derivative and optionally pharmaceutically acceptable diluents and/or adjuvants, and
b) wet milling the at least one antifungal azole derivative and the optionally pharmaceutically acceptable diluents and/or adjuvants using a pearl mill and smooth milling beads composed of at least one organic material.

31. The method of claim 30,
**characterized in that**
wet milling is carried out for about 60 minutes to about 360 minutes, preferably for about 120 minutes to about 240 minutes.
